# NEUE EUROPÄISCHE PATENTSCHRIFT

(11) **EP 1 952 752 B2**
(45) Veröffentlichungstag und Bekanntmachung des Hinweises auf die Entscheidung über den Einspruch: **16.10.2019**
(45) Hinweis auf die Patenterteilung: 27.07.2016
(21) Anmeldenummer: 07002065.6
(22) Anmeldetag: 31.01.2007
(51) Int. Cl.: A61B 1/04

(54) **Endoskopsystem**
Endoscope system
Système d'endoscope

(43) Veröffentlichungstag der Anmeldung: 06.08.2008
(73) Patentinhaber: Richard Wolf GmbH, 75438 Knittlingen (DE)
(72) Erfinder: Heckele, Helmut, 75438 Knittlingen (DE); Völlinger, Hubert, 76437 Rastatt (DE)
(74) Vertreter: Patentanwälte Vollmann Hemmer Lindfeld Partnerschaft mbB

(56) Entgegenhaltungen:
- EP-A1- 1 009 984
- EP-A1- 1 152 261
- EP-A1- 1 622 200
- EP-A2- 1 452 125
- WO-A1-2005/077272
- WO-A2-2005/010799
- DE-A1-102006 017 003
- US-B1- 6 294 775
- ROBERT LANGE ET AL: "Solid-State Time-of-Flight Range Camera" IEEE JOURNAL OF QUANTUM ELECTRONICS, IEEE SERVICE CENTER, PISCATAWAY, NJ, US, Bd. 37, Nr. 3, März 2001 (2001-03), XP011052593 ISSN: 0018-9197
- KURT HÖLLER: "A Novel Approach for 3-D-Video-Endoscopy", 2ND RUSSIAN BAVARIAN CONFERENCE ON BIO-MEDICAL ENGINEERING, 14 June 2006, 14 June 2006 (2006-06-14), pages 1-38, XP055423660, MOSCOW
- S. KRÜGER ET AL: "Neue Techniken der 3-D Endoskopie in der minimal invasiven Chirurgie", CURAC '06 12-14 OKTOBER 2006, XP055422431, Hannover Retrieved from the Internet: URL:https://www.curac.org/vergangene_jahre stagungen/curac06/download/abstracts/124.p df

## Beschreibung

Die Erfindung betrifft ein Endoskopsystem zum Vermessen und/ oder Abbilden dreidimensionaler Strukturen.

Zur Durchführung visueller Untersuchen von menschlichen, tierischen und technischen Körperhöhlen werden Endoskope verwendet, mit denen eine dreidimensionale Betrachtung von Gegenständen möglich ist. Videoendoskope oder Videokameras, die gemeinsam mit einem Endoskop verwendet werden, ermöglichen dagegen lediglich eine zweidimensionale Betrachtung. Bei vielen Anwendung ist es wünschenswert, nicht nur ein zweidimensionales Abbild zu sehen, sondern beispielsweise auch dreidimensionale Strukturen eines zu untersuchenden Objektes darstellen zu können.

US 6,294,775 B1 offenbart ein Fiberoptik-Bildaufnahmesystem für ein Endoskop. Zur Abtastung des Bildfeldes wird dabei eine Lichtleitfaser mit einer Resonanzfrequenz beaufschlagt und so in Schwingung versetzt, dass durch die Bewegung das Bildfeld gescannt wird. Zur Erzeugung eines räumlichen Bildes kann die Entfernung des betrachteten Gegenstandes über eine Messung der Phasenverschiebung des ausgesendeten und des reflektierten Lichtes bestimmt werden. Das Abscannen mittels der vibrierenden Faser ist recht aufwendig, da mechanische Einrichtungen erforderlich sind, um die Faser in Schwingung zu versetzen. Ferner ist die Signalauswertung aufwendig.

Es ist Aufgabe der Erfindung, ein verbessertes endoskopisches System bereitzustellen, mit welchem sich auf einfache Weise Strukturen dreidimensional erfassen und abbilden lassen. Dabei ist es insbesondere erstrebenswert, ein System bereitzustellen, welches in herkömmliche Systeme integriert werden kann bzw. mit dem bestehenden Endoskope ergänzt werden können.

Diese Aufgabe wird durch ein Endoskopsystem mit den in Anspruch 1 angegebenen Merkmalen gelöst. Bevorzugte Ausführungsformen ergeben sich aus den Unteransprüchen, der nachfolgenden Beschreibung sowie den Figuren.

Das erfindungsgemäße Endoskopsystem weist neben den üblichen Bestandteilen eines Endoskops erfindungsgemäß eine Strahlenquelle auf, welche eine modulierte Strahlung aussendet. Diese Strahlenquelle ist in dem Endoskopsystem derart angeordnet, dass ein zu untersuchendes Objekt mit der Strahlung beleuchtbar ist. Dazu ist die Strahlenquelle entweder am distalen Ende des Endoskops angeordnet oder jedoch weiter proximalwärts oder außerhalb des Endoskops, wobei in letzteren Fällen dann ein Wellenleiter zum Leiten der Strahlung zum distalen Ende des Endoskops hin vorgesehen sein muss. Auf jeden Fall tritt die Strahlung am distalen Ende des Endoskops aus und bestrahlt das zu untersuchende Objekt bzw. einen Bereich des zu untersuchenden Objektes.

Erfindungsgemäß weist das Endoskopsystem ferner einen Sensor mit mehreren Sensorelementen auf. Der Sensor bzw. die Sensorelemente dienen zum Erfassen der modulierten Strahlung, welche von dem Objekt reflektiert wird. Dabei ist durch mehrere Sensorelemente vorgesehen, die reflektierte Strahlung an verschiedenen Bildpunkten zu erfassen. Diese entsprechen verschiedenen Punkten des Objektes.

Erfindungsgemäß ist ferner eine Auswerteeinrichtung vorgesehen, welche die von dem Sensor aufgrund der erfassten reflektierten Strahlung generierten Messwerte erhält und basierend auf diesen Messwerten die Entfernung des Objektes von dem Sensor errechnet. Dabei werden insbesondere einzelnen Bildpunkten entsprechende Punkte des Objektes vermessen, d. h. die Entfernung dieser Punkte von dem Sensor bestimmt. Die Entfernungsmessung erfolgt in der Auswerteeinrichtung derart, dass insbesondere in Kenntnis der Modulation der Strahlenquelle aus den Messwerten an den einzelnen Sensorelementen Phasendifferenzen in der Strahlung ermittelt werden. Aus diesen kann dann auf die Signallaufzeit zwischen der Strahlenquelle und den einzelnen Sensor-richtung kann in das Ergänzungsmodul integriert sein, ist jedoch vorzugsweise in eine Steuereinrichtung integriert, welche mit dem Ergänzungsmodul, das direkt an das Endoskop angesetzt werden kann, verbunden ist.

Alternativ ist es möglich, die Sensorik zur dreidimensionalen Erfassung von Objekten direkt in ein Endoskop oder in eine Endoskopoptik zu integrieren, welche dann in einen Endoskopschaft, insbesondere eines herkömmlichen Endoskops, eingesetzt werden kann. Auch auf diese Weise ist es möglich, vorhandene Endoskope nachzurüsten, indem lediglich die Endoskopoptik ausgetauscht wird. Bei dieser Ausführungsform sind in die Endoskopoptik im Wesentlichen diejenigen Elemente integriert, welche vorangehend anhand des Ergänzungsmoduls beschrieben wurden. Ferner ist eine Strahlenquelle vorgesehen, welche eine modulierte Strahlung, insbesondere moduliertes Licht aussendet und so mit dem Endoskop verbindbar ist, dass ein mittels des Endoskops zu untersuchendes Objekt mit der Strahlung beleuchtbar ist. Hierzu ist vorzugsweise eine Anschlusseinrichtung bzw. ein Anschlussstecker vorgesehen, welcher mit dem Lichtleitkabelanschluss eines Endoskops verbindbar ist, wobei in der oben beschriebenen Weise über den Anschlussstecker sowohl die modulierte Strahlung als auch das Licht zur Beleuchtung des Objektes in den Lichtleiter des Endoskops eingekoppelt werden können.

Ferner ist in die Endoskopoptik ein Sensor zum Erfassen der von dem Objekt reflektierten Strahlung an mehreren Bildpunkten integriert, wobei die Erfassung der Strahlung in der oben beschriebenen Weise erfolgen kann. Der Sensor kann bei der vorerwähnten Ausführung vorzugsweise proximalseitig im Endoskop angeordnet sein. Jedoch ist auch eine Anordnung am distalen Ende denkbar. Bei Anordnung am proximalen Ende eines optischen Systems kann auch eine Trenn- oder Umschalteinrichtung vorgesehen sein, um das Signal für die dreidimenter Weise ein Bild des zu untersuchenden Objektes vom distalen Ende des Endoskops zum proximalen Ende des Endoskops zu übertragen, wo beispielsweise eine Videokamera oder ein Okular zur visuellen Betrachtung des Objektes angeordnet sein kann. Erfindungsgemäß wird das optische System jedoch zum anderen auch dazu genutzt, die von dem Objekt reflektierte modulierte Strahlung vom distalen Ende zum proximalen Ende des Endoskops zu leiten. Am proximalen Ende kann dann der Sensor zum Erfassen und Auswerten der reflektierten Strahlung angeordnet sein.

Ferner weist das Endoskopsystem vorzugsweise ein Endoskop mit einem Lichtleiter auf, welcher zur Übertragung von Licht von einer proximalseitig angeordneten oder externen Lichtquelle zum distalen Ende ausgebildet ist, wobei das Licht zur Beleuchtung eines durch die Endoskopoptik zu beobachtenden Objektes vorgesehen ist. Des Weiteren kann im oder am distalen Ende des Endoskops eine entsprechende Lichtquelle angeordnet sein. Am distalen Ende des Endoskops tritt das Licht aus und beleuchtet den Bereich, der durch die Endoskopoptik bzw. das optische System beobachtbar ist.

Besonders bevorzugt kann der Lichtleiter zusätzlich dazu genutzt werden, um die modulierte Strahlung vom proximalen zum distalen Ende des Endoskops zu übertragen, wobei die Strahlung dann am distalen Ende austritt. Diese Ausgestaltung ermöglicht es, die modulierte Strahlung vorzugsweise ohne zusätzliche Elemente durch das Endoskop bzw. durch den Endoskopschaft zu leiten, wenn die Strahlenquelle nicht direkt am distalen Ende des Endoskops angeordnet ist.

Die Beleuchtung von visuell zu betrachtenden und darzustellenden dreidimensionalen Strukturen erfolgt vorzugsweise mittels einer Strahlen- bzw. Lichtquelle, die moduliertes Licht bevorzugt im sichtbaren Bereich aussendet. Dies ermöglicht es, die Optiken bzw. optischen Systeme und Lichtleiter in herkömmlichen Endoskopen zur Übertragung der modulierten Strahlung zu nutzen. So wird es insbesondere ermöglicht, das erfindungsgemäße Messsystem zur dreidimensionalen Erfassung von Objekten in herkömmliche Endoskope zu integrieren bzw. bei herkömmlichen Endoskopen nachzurüsten.

Diese modulierte Strahlenquelle ist weiter zum Aussenden von gepulstem Licht ausgebildet. D. h. das von der Strahlenquelle ausgesendete Licht wird in einem vorgegebenen Takt gepulst. Das modulierte Licht kann dadurch beispielsweise für jene Zeitspannen, in welchen der Videosensor genutzt wird, inaktiv geschaltet werden und somit unerwünschten Einflüssen auf die Farbwahrnehmung des Systems entgegenwirken.

Vorzugsweise ist dazu ein Taktgenerator zur Steuerung der Strahlenquelle und des Sensors und der Auswerteeinrichtung vorgesehen. Der Taktgenerator gibt zum einen die Modulation und die Pulsfrequenz des ausgesendeten Lichtes vor. Zum anderen gibt der Taktgeber auch den Takt für die Erfassung der reflektierten Strahlung an dem Sensor und die nachfolgende Auswertung vor. So ist es zur Erfassung von Phasendifferenzen für die Auswerteeinrichtung erforderlich, den ursprünglichen Phasenverlauf der von der Strahlenquelle ausgesandten Strahlung zu kennen, um dann aus dem Messergebnis des Sensors bzw. der Sensorelemente die Phasendifferenzen bestimmen zu können.

Erfindungsgemäß ist eine gemeinsame Optik zur Übertragung eines Bildes des zu untersuchenden Objektes sowie der von dem Objekt reflektierten modulierten Strahlung in dem Endoskop vorgesehen, wobei ausgangsseitig der gemeinsamen Optik eine Trenn- oder Umschalteinrichtung angeordnet ist, welche die reflektierte modulierte Strahlung dem Sensor zuführt und das Bild einem Okular oder einem Videosensor zuführt. Die Trenn- oder Umschalteinrichtung dient somit dazu, das normale Bild von der reflektierten modulierten Strahlung zu trennen, sodass beide Signale unterschiedlichen Erfassungssystemen zugeführt werden können, nämlich die modulierte Strahlung dem Sensor und die normalen Bildsignale einem Videosensor oder einem Okular für die direkte Betrachtung.

Alternativ ist es möglich, eine Umschalteinrichtung vorzusehen, welche lediglich für die Messung durch den Sensor das Bild bzw. die Strahlung aus der gemeinsamen Optik kurzzeitig dem Sensor zuführt. Diese Umschalteinrichtung wird vorzugsweise sinngemäß durch den Taktgenerator der Strahlenquelle gesteuert. In diesem Fall wird für den Augenblick der Messung das normale Bild, welches an einem Okular sichtbar ist oder von einem Videosensor erfasst wird, kurzzeitig unterbrochen. Vorteilhaft kann die Messung über den Sensor jedoch derart schnell erfolgen, dass diese Unterbrechung für das menschliche Auge nicht sichtbar ist und für den Betrachter ein quasi kontinuierliches Bild zur Verfügung gestellt werden kann.

Gemäß einer weiteren bevorzugten Ausführungsform kann der erfindungsgemäße Sensor Teil eines Videosensors für die Bilderfassung sein bzw. eine bauliche Einheit mit diesem bilden. Das heißt der Sensor kann in einen normalen Videosensor, welcher zur Erfassung des in bekannter Weise von der Endoskopoptik übertragenen Bildes dient, integriert werden. Hierzu können beispielsweise an einzelnen Bildpunkten, besonders bevorzugt an allen Bildpunkten wie bei einer TOF-Kamera Speicherzellen mit Schaltern angeordnet werden, welche es ermöglichen, die von den einzelnen Sensorzellen bzw. Sensorelementen erfassten Intensitäten in den Speicherzellen zu speichern und anschließend für die weitere Berechnung auszulesen. Bei einer solchen Ausgestaltung kann auf eine Teil- oder Umlenkeinrichtung verzichtet werden und es ist sehr einfach möglich das erfindungsgemäße Messsystem in die normale Bildübertragung bzw. Bildverarbeitung eines Endoskops mit Hilfe der Videotechnik zu integrieren.

Eine weitere mögliche Ausführungsform der Erfindung sieht vor, dass die Sensoreinrichtung zum Erfassen der reflektierten modulierten Strahlung sowie eine Optik oder ein Videosensor zur Bildbetrachtung in einem gemeinsamen Modul integriert sind, welches über eine Schnittstelle proximalseitig an dem Endoskopschaft angesetzt ist. Dabei kann die Schnittstelle derart ausgebildet sein, dass es möglich ist, das Modul an herkömmliche Endoskope bzw. Endoskopschäfte proximalseitig anzusetzen, sodass das Messsystem sehr leicht in herkömmliche Endoskope integrierbar ist. Es muss dann idealerweise lediglich das proximalseitig an das Endoskop angesetzte Video-Bilderfassungssystem durch das beschriebene Modul ersetzt werden, welches die Bilderfassung sowie die beschriebene Messtechnik zur dreidimensionalen Erfassung des Objektes integriert.

Weiter bevorzugt ist erfindungsgemäß ein Beleuchtungsanschluss vorgesehen, welcher mit einer Lichtquelle zur Objektbeleuchtung und mit der Strahlenquelle, welche die modulierte Strahlung abgibt, verbunden ist. Der Beleuchtungsanschluss kann z.B. ausgangsseitig als Anschlussstecker ausgebildet sein, welcher mit einem Lichtleitkabelanschluss an dem Endoskopschaft verbunden ist. Das heißt, der Beleuchtungsanschluss dient dazu, die Strahlung bzw. das Licht von der Strahlenquelle in den Beleuchtungspfad des Endoskops, welcher das Licht zur Objektbeleuchtung zum distalen Ende des Endoskops überträgt, einzukoppeln. Diese Einkoppelstelle liegt in dem beschriebenen Beleuchtungsanschluss, welcher eingangsseitig mit zwei Strahlen- bzw. Lichtquellen, nämlich mit der Lichtquelle zur Beleuchtung und der beschriebenen Strahlenquelle für die modulierte Strahlung verbindbar ist. Ausgangsseitig ist nur ein Ausgang vorgesehen, welcher vorzugsweise als Standardanschlussstecker bzw. -kupplung ausgebildet ist, welche sich mit den üblichen Lichtleitkabelanschlüssen von Endoskopschäften verbinden lässt. Auf diese Weise wird es einfach möglich, herkömmliche Endoskope auch für das erfindungsgemäße Messsystem zu verwenden und die erforderliche modulierte Strahlung in herkömmliche Endoskope einzukoppeln.

Wie bereits in der vorangehenden Beschreibung erwähnt, ist es besonders bevorzugt, bestehende oder herkömmliche Endoskope derart nachrüsten zu können, dass mit diesen eine dreidimensionale Erfassung bzw. Vermessung der zu untersuchenden Objekte möglich wird. Hierzu ist ein Ergänzungsmodul für ein Endoskop vorgesehen, welches sich als Zubehörteil an Endoskope ansetzen bzw. in Endoskopsysteme integrieren lässt.

Dazu weist das Ergänzungsmodul eine Schnittstelle auf, über welche es an ein Endoskop angesetzt und mit diesem verbunden werden kann. Die Schnittstelle ist dazu vorzugsweise als Standardschnittstelle ausgebildet, um mit herkömmlichen Endoskoptypen bzw. Typen von Endoskopoptiken verbunden werden zu können. In dem Ergänzungsmodul ist eine Bildbetrachtungseinrichtung angeordnet, über welche in herkömmlicher Weise das Bild des im Blickfeld des Endoskops gelegenen Objektbereiches erfasst werden kann. Hierzu kann ein Okular oder ein Videosensor mit einem sich daran anschließenden Videosystem vorgesehen sein. Ferner ist vorzugsweise in dem Ergänzungsmodul ein Anschluss für eine externe Lichtquelle vorgesehen, welche die übliche Beleuchtung des distalseitigen Bereiches des Endoskops bewirkt. Hierzu wird das Licht von der Lichtquelle z.B. durch das Ergänzungsmodul zu einem Anschlussstecker geleitet und über diesen in den Lichtleiter des Endoskops eingeleitet. Alternativ kann die Lichtquelle auch in das Ergänzungsmodul integriert sein.

In dem Ergänzungsmodul ist ferner eine Strahlenquelle, besonders bevorzugt ebenfalls eine Lichtquelle, vorgesehen, welche eine modulierte Strahlung aussendet und vorzugsweise ebenfalls mit einem oder dem zuvor genannten Anschlussstecker verbunden ist. Das heißt, die von der Strahlenquelle abgegebene modulierte Strahlung wird über den Anschlussstecker ebenfalls in den Lichtleiter des Endoskops eingeleitet, sodass die modulierte Strahlung bevorzugt gemeinsam mit dem Licht zur Beleuchtung distalseitig aus dem Endoskop austritt.

Darüber hinaus weist das Ergänzungsmodul einen Sensor auf, wobei der Sensor über die Endoskop-Ergänzungsmodul-Schnittstelle modulierte Strahlung bzw. moduliertes Licht empfängt, welches von einem mit dem Endoskop zu untersuchenden Objekt reflektiert wurde. Das heißt die modulierte Strahlung wird durch den Lichtleiter zum distalen Ende des Endoskops geführt, tritt dort aus und trifft auf das Objekt. Von dem Objekt wird die Strahlung reflektiert und tritt in das distalseitige Ende der Endoskopoptik ein, welche die Strahlung zum proximalen Ende und zu der Schnittstelle zu dem Ergänzungsmodul hin leitet. Der Sensor ist zum Erfassen der von dem Objekt reflektierten Strahlung an einem oder mehreren Bildpunkten in der oben anhand des Endoskops beschriebenen Weise ausgebildet und weist dazu vorzugsweise mehrere Sensorelemente auf.

Der Sensor ist mit einer Auswerteeinrichtung verbunden, welche von dem Sensor Messwerte der erfassten Strahlung erhält. Basierend auf diesen Messwerten und insbesondere in Kenntnis der Modulation der Strahlenquelle bestimmt die Auswerteeinrichtung Phasendifferenzen an dem einen oder den mehreren Bildpunkten und errechnet auf deren Grundlage die Entfernung des Objektes von der Sensoreinrichtung. Die Auswertung der von dem Sensor erfassten Strahlung erfolgt in der oben anhand des Endoskopsystems beschriebenen Weise. Die Auswerteeinrichtung kann in das Ergänzungsmodul integriert sein, ist jedoch vorzugsweise in eine Steuereinrichtung integriert, welche mit dem Ergänzungsmodul, das direkt an das Endoskop angesetzt werden kann, verbunden ist.

Alternativ ist es möglich, die Sensorik zur dreidimensionalen Erfassung von Objekten direkt in ein Endoskop oder in eine Endoskopoptik zu integrieren, welche dann in einen Endoskopschaft, insbesondere eines herkömmlichen Endoskops, eingesetzt werden kann. Auch auf diese Weise ist es möglich, vorhandene Endoskope nachzurüsten, indem lediglich die Endoskopoptik ausgetauscht wird. Bei dieser Ausführungsform sind in die Endoskopoptik im Wesentlichen diejenigen Elemente integriert, welche vorangehend anhand des Ergänzungsmoduls beschrieben wurden. Ferner ist eine Strahlenquelle vorgesehen, welche eine modulierte Strahlung, insbesondere moduliertes Licht aussendet und so mit dem Endoskop verbindbar ist, dass ein mittels des Endoskops zu untersuchendes Objekt mit der Strahlung beleuchtbar ist. Hierzu ist vorzugsweise eine Anschlusseinrichtung bzw. ein Anschlussstecker vorgesehen, welcher mit dem Lichtleitkabelanschluss eines Endoskops verbindbar ist, wobei in der oben beschriebenen Weise über den Anschlussstecker sowohl die modulierte Strahlung als auch das Licht zur Beleuchtung des Objektes in den Lichtleiter des Endoskops eingekoppelt werden können.

Ferner ist in die Endoskopoptik ein Sensor zum Erfassen der von dem Objekt reflektierten Strahlung an einem oder mehreren Bildpunkten integriert, wobei die Erfassung der Strahlung in der oben beschriebenen Weise erfolgen kann. Der Sensor kann bei der vorerwähnten Ausführung vorzugsweise proximalseitig im Endoskop angeordnet sein. Jedoch ist auch eine Anordnung am distalen Ende denkbar. Bei Anordnung am proximalen Ende eines optischen Systems kann auch eine Trenn- oder Umschalteinrichtung vorgesehen sein, um das Signal für die dreidimensionale Vermessung von dem üblichen Bildsignal trennen zu können. Ferner ist für die Endoskopoptik die bereits beschriebene Auswerteeinrichtung vorgesehen, welche von dem Sensor Messwerte der erfassten Strahlung erhält und basierend auf diesen Messwerten und insbesondere den Kenntnissen der Modulation der Strahlenquelle an dem einen oder den mehreren Bildpunkten Phasendifferenzen bestimmt und auf deren Grundlage die Entfernung des Objektes von dem Sensor errechnet. Diese Auswerteeinrichtung kann entweder direkt in die Endoskopoptik integriert sein oder er ist in eine Steuereinrichtung integriert, welche mit der Endoskopoptik und der an dieser angeordneten Sensoreinrichtung über eine Leitung verbunden ist.

Es ist zu verstehen, dass im Zusammenhang mit der beschriebenen Endoskopoptik und dem beschriebenen Ergänzungsmodul auch die oben anhand des Endoskopsystems beschriebenen bevorzugten Merkmale verwirklicht werden können.

Nachfolgend wird die Erfindung beispielhaft anhand der beigefügten Figuren beschrieben. In diesen zeigt:
- Figur 1 und 2: schematische Darstellungen des erfindungsgemäßen Endoskopsystems
- Figur 3: schematisch die Funktionsweise einer TOF-Kamera
- Figuren 4 bis 7: vier verschiedene Möglichkeiten der Bildteilung.

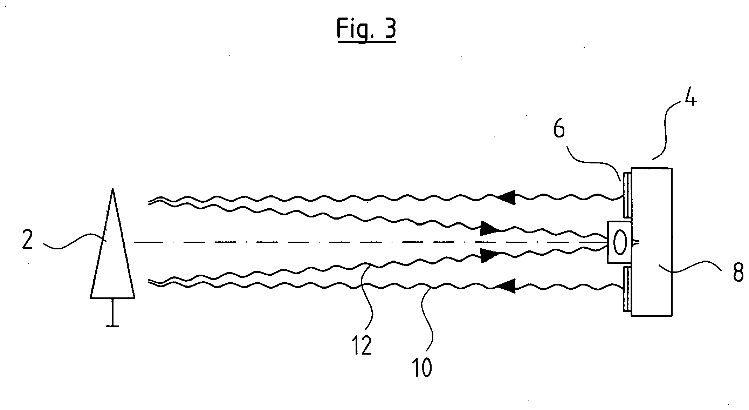

Bei dem erfindungsgemäßen Endoskopsystem wird zur dreidimensionalen Vermessung des zu untersuchenden Objektes ein Messprinzip eingesetzt, welches einer TOF ("time of flight") Kamera entspricht. Die Funktionsweise dieses Kamerasystems wird anhand von Figur 3 erläutert. Dort ist ein Objekt 2 gezeigt, welches mittels der Kamera 4 beobachtet bzw. vermessen wird. Die Kamera 4 weist eine Beleuchtungseinrichtung 6 und einen Sensor 8 auf, wobei die Beleuchtungseinrichtung 6 seitlich des Sensors 8 im Wesentlichen in einer Ebene mit diesem platziert ist. Die Beleuchtungseinrichtung 6 ist so ausgebildet, dass sie moduliertes, d. h. insbesondere gepulstes Licht 10 (modulierte Strahlung) aussendet. Bei Verwendung in einem Endoskop liegt das gepulste Licht 10 vorzugsweise im sichtbaren Bereich, sodass es über die üblichen optischen Elemente in dem Endoskop übertragbar ist.

Das gepulste Licht 10 trifft auf das Objekt 2 auf und wird von diesem als reflektiertes Licht 12 zu der Kamera 4 zurückgesendet und dort von dem Sensor 8 erfasst, der eine Vielzahl von Sensorelementen aufweist, von welchen jedes einem einzelnen Bildpunkt oder einer bestimmten Gruppe von Bildpunkten zugeordnet ist, sodass vorzugsweise im Wesentlichen das gesamte Objekt 2 durch einzelne Bildpunkte erfasst wird.

Aufgrund der dreidimensionalen Struktur des Objektes 2 sind die der Kamera 4 zugewandten Oberflächenbereiche des Objektes 2 unterschiedlich weit von der Kamera 4 beabstandet. Dies führt zu unterschiedlichen Laufzeiten des Lichtes von der Beleuchtungseinrichtung 6 zurück zu dem Sensor 8. Für die Bildpunkte, bei denen das Objekt 2 bzw. dessen Oberfläche weiter von der Kamera 4 entfernt ist, ergeben sich längere Laufzeiten des Lichtes. Die Laufzeitunterschiede führen aufgrund der Modulation des ausgesendeten Lichtes 6 zu Phasendifferenzen, die erfasst und ausgewertet werden, um die Laufzeit und damit die Entfernung der Bildpunkte zu bestimmen. Hieraus wiederum lässt sich dann die dreidimensionale Struktur des Objektes 2 ableiten. Die Funktionsweise einer solchen TOF-Kamera ist beispielsweise aus DE 4 440 613 C1 oder EP 1 622 200 A1 bekannt.

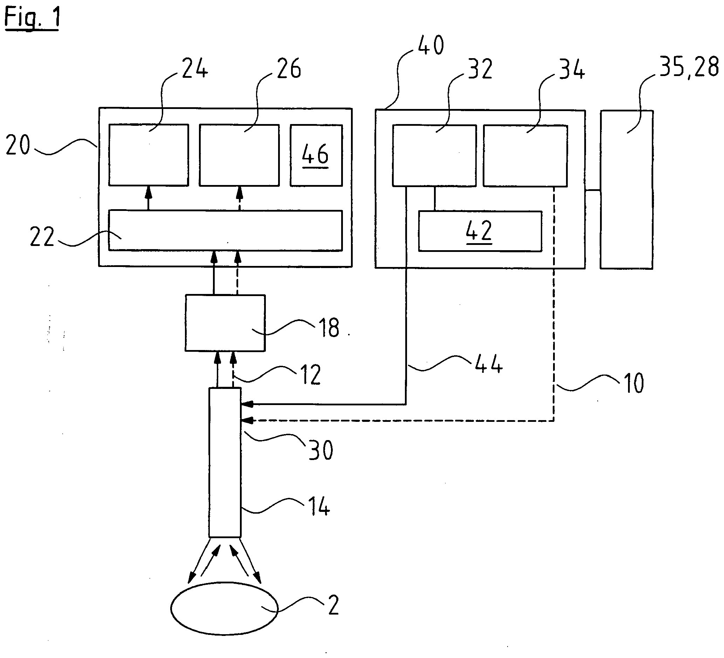

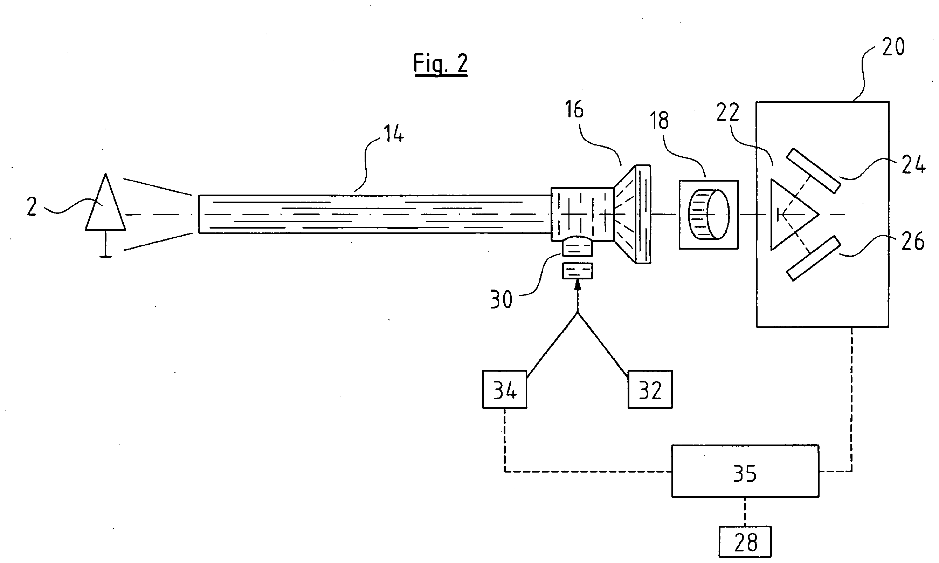

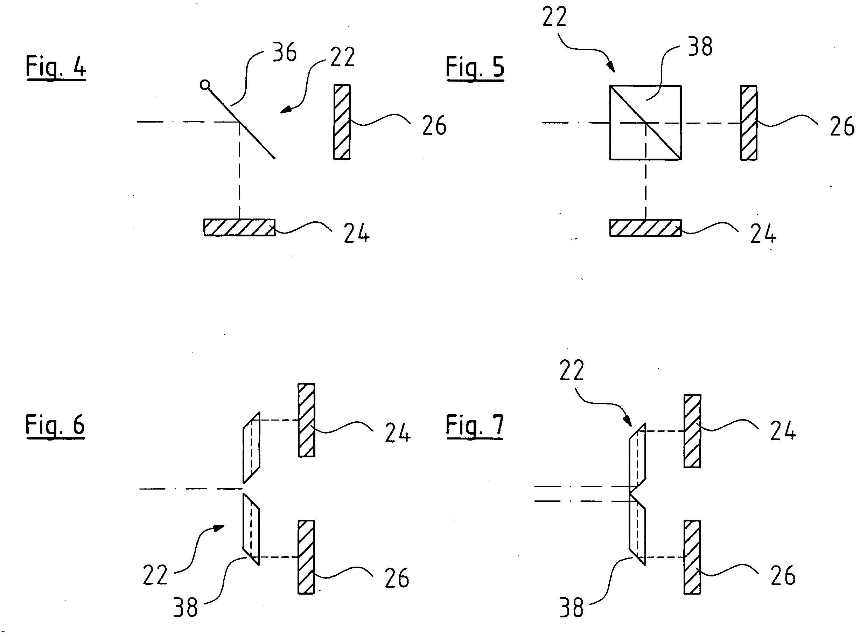

Erfindungsgemäß wird dieses von den vorerwähnten TOF-Kameras angewandte Messprinzip mit einem Endoskop 2 kombiniert, was schematisch in Figuren 1 und 2 dargestellt ist. Dort wird ein Endoskop 14 verwendet, um das Objekt 2 zu betrachten bzw. zu untersuchen. Dabei ist das Endoskop 14 in herkömmlicher Weise ausgestaltet, d. h. es weist in seinem Inneren insbesondere zumindest ein optisches System zur Betrachtung des Objektes 2 und einen Lichtleiter zur Beleuchtung des Objektes 2 auf. Proximalseitig weist das Endoskop 14 eine Aufnahme 16 auf, an welcher ein Objektiv 18 sowie weiter proximalseitig anschließend eine Sensoreinrichtung 20 angesetzt wird. Dabei können das Objektiv 18 und die Sensoreinrichtung 20 in einem Modul, vorzugsweise Ergänzungsmodul zusammengefasst sein. In der Sensoreinrichtung 20 ist ein Bildteiler 22 vorgesehen, welcher das von dem Objektiv 18 übertragene Bild auf bspw. zwei Sensoren 24 und 26 z. B. gemäß der anhand von Figuren 4 bis 7 beschriebenen Weisen aufteilt. Der Sensor 24 ist als herkömmlicher Videosensor ausgebildet, welcher das durch das Endoskop 14 übertragene Bild des Objektes 2 erfasst, was dann auf einem Monitor 28 zur Anzeige gebracht werden kann. Der Sensor 26 bildet eine Sensoreinrichtung zum Erfassen der dreidimensionalen Struktur des Objektes 2 bzw. zu dessen Vermessung und entspricht der vorangehend beschriebenen TOF-Kamera 4.

Das Endoskop 14 weist in der Nähe seines proximalen Endes in üblicher Weise einen Lichtleitkabelanschluss 30 auf, welcher mit dem proximalen Ende eines Lichtleiters im Inneren des Endoskops 14 verbunden ist, welcher am distalen Ende endet. Der Lichtleitkabelanschluss 30 wird erfindungsgemäß zum einen mit einer herkömmlichen Beleuchtungseinrichtung 32 verbunden, welche konstant Licht zur Beleuchtung des Objektes 2 aussendet, um auf dem Sensor 24 ein Bild zu erzeugen. Ferner ist eine modulierende Strahlen- bzw. Lichtquelle 34 vorgesehen, welche moduliertes bzw. gepulstes Licht aussendet, das ebenfalls in den Lichtleitkabelanschluss 30 eingespeist wird. Vorzugsweise wird das von der Beleuchtungseinrichtung 32 sowie der Strahlenquelle 34 ausgesendete Licht in einem Anschlussbauteil mit Hilfe bekannter optischer Elemente zusammengeführt, sodass das Anschlussbauteil dann nur einen Ausgang aufweist, welcher mit dem Lichtleitkabelanschluss 30 verbunden wird.

Die Strahlenquelle 34 sowie die Sensoreinrichtung 20 sind mit einer Steuereinrichtung 35 verbunden. Die Steuereinrichtung 35 weist einen Taktgenerator auf, welcher den Takt für die Pulsfrequenz und Pulslängen des von der Strahlenquelle 34 ausgesendeten Lichtes vorgibt. Ferner erfasst die Steuereinrichtung 35 die von dem Sensor 26 erfassten Bildsignale und wertet diese als Auswerteeinheit aus. Der Steuereinrichtung 35 ist der Takt des von der Strahlenquelle 34 ausgesendeten Lichtes bekannt, sodass es dann beim Erfassen des reflektierten Lichtes aufgrund der Phasendifferenzen die Laufzeiten für einzelne Bildpunkte bestimmen und auf diese Weise die dreidimensionale Struktur des Objektes 2 errechnen kann. Diese wird dann ebenfalls auf dem Monitor 28 zur Ansicht gebracht. Alternativ kann auch ein separater Monitor vorgesehen sein. Die Bildverarbeitung für das von dem Sensor 24 aufgenommene Bild kann ebenfalls in die Steuereinrichtung 35 integriert sein. Alternativ kann hier ein separates Bildverarbeitungssystem vorgesehen sein.

In dem in Figur 2 gezeigten Beispiel sind zwei Sensoren 24 und 26 vorgesehen. Bevorzugt lassen sich beide Funktionen auch in einen Sensor integrieren, sodass auf die Bildverteilungseinrichtung 22 verzichtet werden könnte.

Wie in Figur 1 gezeigt, besteht das Endoskop bzw. System neben dem eigentlichen Endoskop 14 mit dem zugehörigen Objektiv 18 im Wesentlichen aus drei Modulen, nämlich der Sensoreinrichtung 20, einem Beleuchtungsmodul 40 sowie aus einem Steuermodul, welches aus der Steuereinrichtung 35 und ggf. dem Monitor 28 gebildet wird. Das Steuermodul kann dabei beispielsweise von einem Personal-Computer als Steuereinrichtung 35 mit zugehörigem Monitor 28 gebildet werden. Das Beleuchtungsmodul 40 umfasst die Beleuchtungseinrichtung 32 sowie die Strahlenquelle 34 und ggf. einen elektronischen Verschluss (shutter) 42 zur Steuerung bzw. zum Ein- und Ausschalten des von der Beleuchtungseinrichtung 32 abgegebenen Lichtes. Die von der Strahlenquelle 34 abgegebene modulierte Strahlung bzw. das gepulste Licht 10 sowie das von der Beleuchtungseinrichtung 32 ausgesendete Licht 44 können über separate Lichtleiter dem Beleuchtungsanschluss 30 des Endoskops 14 zugeführt werden. Alternativ kann ein gemeinsamer Lichtleiter vorgesehen sein, welcher das Beleuchtungsmodul 40 mit dem Lichtleitkabelanschluss 30 verbindet, d. h. das gepulste Licht 10 und das Licht 44 werden bereits im Beleuchtungsmodul 40 in einen gemeinsamen Lichtpfad zusammengeführt.

In der Sensoreinrichtung 20 sind die Sensoren 24 und 26 sowie ggf. eine Bildteiler 22 sowie erforderliche elektronische Bauteile 46 zur Steuerung bzw. Regelung der vorgenannten Komponenten zusammengefasst.

Die Module Sensoreinrichtung 20, Beleuchtungsmodul 40 sowie die Steuereinrichtung 35 können als Zubehörkomponenten zur Verwendung mit einem herkömmlichen Endoskop 14 vorgesehen sein. So lässt sich ein herkömmliches Endoskop 14 zu einem Endoskop, welches die dreidimensionale Erfassung von Strukturen ermöglicht, aufrüsten.

Der mögliche Aufbau und die mögliche Funktion eines Bildteilers 22 wird anhand der Figuren 4 bis 7 erläutert, welche vier mögliche Ausführungsbeispiele zeigen.

Figur 4 zeigt einen Bildteiler, welcher einen Schwenkspiegel 36 aufweist, welcher in der gezeigten Stellung das Bild, welches proximalseitig aus dem optisches System des Endoskops 14 bzw. dem Objektiv 18 austritt zu dem Sensor 24 leitet. Für die Messung mittels des Sensors 26 wird der Spiegel 36 in Richtung des Pfeils in Figur 4 verschwenkt, sodass der Strahlengang nicht mehr auf den Sensor 24, sondern auf den Sensor 26 trifft und dieser die reflektierte modulierte Strahlung für die beschriebene Messung empfängt. Für den Augenblick der Messung empfängt der Sensor 24 somit kein Bild mehr. Allerdings kann die Messung so schnell erfolgen, dass der Spiegel 36 nur sehr kurzzeitig in die Position verschwenkt werden muss, in der das Bild auf den Sensor 26 gelenkt wird. Dadurch kann die Unterbrechung des Bildes für den Sensor 24 so kurz gehalten werden, dass sie für das menschliche Auge nicht sichtbar ist. Alternativ könnten die Sensoren 24 und 26 auch umgekehrt angeordnet werden.

Figur 5 zeigt einen Strahlteiler 38, durch welchen das aus dem Endoskop 14 proximalseitig austretende Lichtbündel geteilt und gleichzeitig zu den Sensoren 24 und 26 gerichtet wird. Dabei erfolgt die Teilung nach den Wellenlängen, sodass diejenigen Wellenlängen, welche denen von der Strahlenquelle 34 ausgesendeten Wellenlängen entsprechen, zu dem Sensor 26 und alle übrigen Wellenlängen zu dem Sensor 24 gelenkt werden. So erfasst nur der Sensor 26 die reflektierte modulierte Strahlung, welche von der Strahlenquelle 34 ausgesandt wurde.

Anstelle einer Strahlteilung aufgrund unterschiedlicher Wellenlängen, ist auch eine geometrische Strahlteilung, wie anhand der Figuren 6 und 7 gezeigt, möglich. Bei dieser wird das aus dem Endoskop 14 proximalseitig austretende Lichtbündel geometrisch vorzugsweise zu gleichen Teilen auf die Sensoren 24 und 26 verteilt, sodass beide Sensoren 24, 26 vollständig das gesamte Bild und das gesamte Wellenlängenspektrum erfassen. Hierbei verringert sich dann jedoch die Intensität des Lichtes an jedem der Sensoren 24 und 26. Figur 6 zeigt eine einkanalige geometrische Strahlteilung, d. h. in dem Endoskop ist nur ein Optikkanal vorgesehen. Figur 7 zeigt eine zweikanalige geometrische Strahlteilung, bei welcher zwei Optikkanäle vorgesehen sind, wobei das Licht jedes der beiden Kanäle auf beide Sensoren 24 und 26 verteilt wird.

### Bezugszeichenliste

- 2: Objekt
- 4: Kamera
- 6: Beleuchtungseinrichtung
- 8: Sensor
- 10: gepulstes Licht
- 12: reflektiertes Licht
- 14: Endoskop
- 16: Aufnahme
- 18: Objektiv
- 20: Sensoreinrichtung
- 22: Bildteiler
- 24, 26: Sensoren
- 28: Monitor
- 30: Lichtleitkabelanschluss
- 32: Beleuchtungseinrichtung
- 34: Strahlenquelle
- 35: Steuereinrichtung
- 36: Schwenkspiegel
- 38: Strahlteiler
- 40: Beleuchtungsmodul
- 42: Verschluss
- 44: Licht
- 46: Elektronische Bauteile

Die Beispiele und Ausführungsformen, die nicht unter den Schutzumfang der beigefügten Ansprüche fallen, dienen lediglich zur Erläuterung und sind nicht Teil der gegenwärtigen Erfindung.

Die Erfindung ist in den folgenden Ansprüchen definiert.

## Patentansprüche

1. Endoskopsystem zum Erfassen dreidimensionaler Strukturen mit mindestens einer Strahlenquelle (34) in Form einer Beleuchtungseinrichtung, welche eine modulierte Strahlung im sichtbaren Bereich aussendet und derart eingesetzt ist, dass ein mittels des Endoskopssystems zu untersuchendes Objekt (2) mit der Strahlung beleuchtbar ist, mindestens einem Sensor (26) mit mehreren Sensorelementen zum Erfassen der von dem Objekt (2) reflektierten Strahlung an verschiedenen Bildpunkten, wobei eine dreidimensionale Struktur des Objektes (2) zu derart unterschiedlichen Laufzeiten der Strahlung von der Beleuchtungseinrichtung (34) zurück zu dem Sensor (26) führt, dass sich für Bildpunkte, an denen das Objekt (2) weiter entfernt ist, längere Laufzeiten der Strahlung ergeben, sowie einer Auswerteeinrichtung (35), welche von dem Sensor (26) Messwerte der erfassten Strahlung erhält und basierend auf diesen Messwerten an den einzelnen Bildpunkten Phasendifferenzen, welche aus den Laufzeitunterschieden der Strahlung resultieren, bestimmt und auf deren Grundlage die Laufzeit und damit die Entfernung von den Bildpunkten entsprechenden Punkten des Objektes (2) von dem Sensor (26) errechnet, wobei das Endoskopsystem ein übliches Videoendoskop zur Übertragung und Darstellung eines Bildes des zu untersuchenden Objektes aufweist, welches es ermöglicht, gleichzeitig während der Vermessung des zu untersuchenden Objektes dieses auf einem Monitor zu betrachten und eine gemeinsame Optik zur Übertragung eines Bildes des zu untersuchenden Objektes (2) sowie der von dem Objekt (2) reflektierten modulierten Strahlung aufweist, wobei ausgangsseitig der gemeinsamen Optik eine Trenn- oder Umschalteinrichtung (22) angeordnet ist, welche die reflektierte modulierte Strahlung dem Sensor (26) und das Bild einem Okular oder einem Videosensor (24) zuführt.

2. Endoskopsystem nach Anspruch 1, bei welchem die Auswerteeinrichtung (35) zur Ausgabe der errechneten Entfernungswerte und insbesondere einer dreidimensionalen Darstellung des Objektes (2) auf einem Monitor (28) ausgebildet ist.

3. Endoskopsystem nach einem der vorangehenden Ansprüche, welches zumindest ein sich vom distalen zum proximalen Ende erstreckendes optisches System aufweist, welcher zur Bildübertragung und zur Übertragung der von dem Objekt (2) reflektierten modulierten Strahlung dient.

4. Endoskopsystem nach einem der vorangehenden Ansprüche, welches zumindest einen Lichtleiter aufweist, welcher zur Übertragung von Licht von einer proximalseitig angeordneten Lichtquelle (32) zum distalen Ende ausgebildet ist, wobei das Licht zur Beleuchtung eines über die Endoskopoptik zu beobachtenden Bereichs vorgesehen ist.

5. Endoskopsystem nach Anspruch 4, bei welchem der zumindest eine Lichtleiter zusätzlich zur Übertragung der modulierten Strahlung zum distalen Ende des Endoskops vorgesehen ist.

6. Endoskopsystem nach einem der vorangehenden Ansprüche, bei welchem die Strahlenquelle (34) zum Aussenden von gepulstem Licht als modulierte Strahlung ausgebildet ist.

7. Endoskopsystem nach einem der vorangehenden Ansprüche, bei welchem ein Taktgenerator zur Steuerung der Strahlenquelle (34) und des Sensors und der Auswerteeinrichtung (35) vorgesehen ist.

8. Endoskopsystem nach einem der vorangehenden Ansprüche, bei welchem der Sensor (26) zur Erfassung der reflektierten modulierten Strahlung sowie eine Optik oder ein Videosensor (24) zur Bildbetrachtung in einem Modul (20) integriert sind, welches über eine Schnittstelle (15) proximalseitig an das Endoskop angesetzt ist.

9. Endoskopsystem nach einem der vorangehenden Ansprüche, bei welchem ein Beleuchtungsanschluss (30) vorgesehen ist, welcher mit einer Lichtquelle (32) zur Objektbeleuchtung und mit der Strahlenquelle (34) verbunden ist.

## Claims

1. An endoscope system for detecting three-dimensional structures, with at least one radiation source (34) in the form of an illumination device which emits modulated radiation in the visible region and is applied in a manner such that an object (2) to be examined by way of the endoscope system can be illuminated with the radiation, with at least one sensor (26) with several sensor elements for detecting the radiation reflected by the object (2) at different picture points, wherein a three-dimensional structure of the object (2) leads to such different time-of-flights of the radiation from the illumination device (34) back to the sensor (26), that longer time-of-flights of the radiation result for picture points, at which the object (2) is distanced further, as well as with an evaluation device (35) which obtains measurement values of the detected radiation from the sensor (26) and determines phase differences on the basis of these measurement values at the individual picture points, said phase differences resulting from the time-of-flight differences of the radiation and on the basis of which the time-of-flight and herewith the distance of the points of the object (2) which correspond to the picture points, to the sensor (26), is computed, wherein the endoscope system comprises a common video endoscope for transmitting and representing a picture of the object to be examined and permitting the object to be examined to be observed on a monitor simultaneously during the measurement of this and comprising common optics for transmitting a picture of the object (2) to be examined, as well as the modulated radiation which is reflected by the object (2), wherein a separating device or switch-over device (22) is arranged at the exit side of the common optics and leads the reflected modulated radiation to the sensor (26), and the picture to the eyepiece or to a video sensor (24).

2. An endoscope system according to claim 1, with which the evaluation device (35) is designed for outputting the computed distance values, and in particular a three-dimensional representation of the object (2) on a monitor (28).

3. An endoscope system according to one of the preceding claims, which comprises at least one optical system extending from the distal to the proximal end, said system serving for the picture transmission and for the transmission of the modulated radiation which is reflected by the object (2).

4. An endoscope system according to one of the preceding claims, which comprises at least one fiber-optic which is designed for the transmission of light from a light source (32) which is arranged at the proximal side, to the distal end, wherein the light is provided for illuminating a region which is to be observed via the endoscope optics.

5. An endoscope system according to claim 4, with which the at least one fiber-optic is additionally provided for transmitting the modulated radiation to the distal end of the endoscope.

6. An endoscope system according to one of the preceding claims, with which the radiation source (34) is designed for emitting pulsed light as modulated radiation.

7. An endoscopic system according to one of the preceding claims, with which a clock generator for controlling the radiation source (34) and the sensor and the evaluation device (35) is provided.

8. An endoscope system according to one of the preceding claims, with which the sensor (26) for detecting the reflected modulated radiation, as well as optics or a video sensor (24) for picture observation are integrated in a module (20), said module being attached to the endoscope on the proximal side via an interface (15).

9. An endoscope system according to one of the preceding claims, with which an illumination connection (30) is provided, said illumination connection being connected to a light source (32) for object illumination and to the radiation source (34).

## Revendications

1. Système d'endoscope pour capter des structures tridimensionnelles, comportant au moins une source de rayonnement (34) sous forme d'un dispositif d'éclairage, qui émet un rayonnement modulé dans le domaine visible et qui est utilisée de façon telle qu'un objet (2) à examiner à l'aide du système d'endoscope puisse être illuminé avec le rayonnement, au moins un capteur (26) ayant plusieurs éléments capteurs pour capter à différents points d'image le rayonnement réfléchi par l'objet (2), une structure tridimensionnelle de l'objet (2) faisant qu'il en découle des temps de propagation du rayonnement pour le retour du dispositif d'éclairage (34) au capteur (26) différents de façon telle que pour des points d'image dont l'objet est plus éloigné, il résulte des temps de propagation plus longues , ainsi qu'un dispositif d'exploitation (35) qui reçoit du capteur (26) des valeurs de mesure du rayonnement capté et détermine aux différents points d'image, sur la base desdites valeurs de mesure, des différences de phase résultant des différences de temps de propagation du rayonnement et calcule, sur la base de celles-ci, le temps de propagation et par conséquent la distance entre des points correspondant aux points d'image de l'objet (2) et le capteur (26), le système d'endoscope comprenant un endoscope vidéo traditionnel pour la transmission et la représentation d'une image de l'objet à examiner qui permet, simultanément au mesurage de l'objet à examiner, d'observer ce dernier sur un moniteur, et une optique commune pour la transmission de l'image de l'objet à examiner (2) ainsi que du rayonnement modulé réfléchi par l'objet (2), un dispositif de séparation et de commutation étant disposé en sortie de l'optique commune, lequel amène le rayonnement modulé réfléchi au capteur (26) et l'image à un oculaire ou un capteur vidéo (24).

2. Système d'endoscope selon la revendication 1, dans lequel le dispositif d'exploitation (35) est conçu pour une édition des valeurs de distance calculées et notamment pour une représentation tridimensionnelle de l'objet (2), sur un moniteur (28).

3. Système d'endoscope selon l'une des revendications précédentes, qui comprend au moins un système optique s'étendant de l'extrémité distale à l'extrémité proximale, lequel sert à la transmission d'image et à la transmission du rayonnement modulé réfléchi par l'objet (2).

4. Système d'endoscope selon l'une des revendications précédentes, qui comprend au moins un guide de lumière qui est conçu pour la transmission de lumière d'une source de lumière (32) disposée du côté proximal vers l'extrémité distale, la lumière étant prévue pour illuminer une zone destinée à être observée via l'optique de l'endoscope.

5. Système d'endoscope selon la revendication 4, dans lequel ledit au moins un guide de lumière est prévu en plus pour la transmission du rayonnement modulé vers l'extrémité distale de l'endoscope.

6. Système d'endoscope selon l'une des revendications précédentes, dans lequel la source de rayonnement (34) est conçue pour émettre de la lumière pulsée comme rayonnement modulé.

7. Système d'endoscope selon l'une des revendications précédentes, dans lequel un générateur d'horloge est prévu pour commander la source de rayonnement (34) et le capteur et le dispositif d'exploitation (35).

8. Système d'endoscope selon l'une des revendications précédentes, dans lequel le capteur (26) pour capter le rayonnement modulé réfléchi ainsi qu'une optique ou un capteur vidéo (24) pour l'observation d'image sont intégrés dans un module (20) qui est appliqué à l'endoscope, du côté proximal, via une interface (15).

9. Système d'endoscope selon l'une des revendications précédentes, dans lequel est prévu un raccord de lumière (30) qui est relié à une source de lumière (32) pour l'illumination de l'objet et à la source de rayonnement (34).
